**Europäisches Patentamt**

(19) **European Patent Office**

**Office européen des brevets**

(11) Veröffentlichungsnummer: **0 053 680**
**B1**

(12) **EUROPÄISCHE PATENTSCHRIFT**

(45) Veröffentlichungstag der Patentschrift:
07.03.84

(51) Int. Cl.³: **A 61 B 3/02,** A 61 B 3/12

(21) Anmeldenummer: **81108496.1**

(22) Anmeldetag: **19.10.81**

(54) Vorrichtung zur subjektiven und objektiven Refraktionsbestimmung.

(30) Priorität: **29.11.80 DE 3045139**

(43) Veröffentlichungstag der Anmeldung:
**16.06.82 Patentblatt 82/24**

(45) Bekanntmachung des Hinweises auf die Patenterteilung:
**07.03.84 Patentblatt 84/10**

(84) Benannte Vertragsstaaten:
**CH DE FR GB LI**

(56) Entgegenhaltungen:
**US - A - 2 798 408**
**US - A - 3 524 702**
**US - A - 3 874 774**
**US - A - 3 927 933**
**US - A - 4 105 302**

(73) Patentinhaber: **Firma Carl Zeiss, Postfach 1369/1380, D-7082 Oberkochen (DE)**

(72) Erfinder: **Lang, Walter, Dr., Finkenweg 33, D-7923 Königsbronn (DE)**
Erfinder: **Muchel, Franz, Dipl.-Phys., Lortzingstrasse 2, D-7923 Königsbronn (DE)**
Erfinder: **Blaha, Erich, Staufenstrasse 15, D-7081 Essingen (DE)**

Vorrichtung zur subjektiven und objektiven Refraktionsbestimmung

Die vorliegende Erfindung betrifft eine Vorrichtung zur subjektiven und objektiven Refraktionsbestimmung.

Es sind, z.B. aus «ABC der Optik» 1961, Seiten 742/743 objektiv arbeitende Augenrefraktometer bekannt, bei denen ein Testobjekt auf die Netzhaut des zu untersuchenden Auges abgebildet und das vom Auge reflektierte Licht beobachtet wird. Ein im Beleuchtungsstrahlengang angeordnetes optisches Element, z.B. eine Linse oder ein Prisma, wird solange verschoben, bis das Testbild auf der Netzhaut scharf erscheint, wobei die Stellung dieses Elementes dann ein Mass für den gesuchten Refraktionswert ist.

Diese bekannten Geräte haben den Nachteil, dass sie keine gleichzeitige subjektive Refraktionsbestimmung ermöglichen und dass nur eine monokulare Bestimmung möglich ist.

Aus der DE-PS 12 99 907 ist ein Gerät zur subjektiven und gleichzeitigen objektiven Refraktionsbestimmung bekannt, bei dem auf eine Bildwand gleichzeitig zwei Testmarken in unterschiedlichen Spektralbereichen projiziert werden und der Proband durch einen Phoropter hindurch diese Bildwand beobachtet. Das von der Netzhaut des Auges reflektierte Licht wird über einen teildurchlässigen Spiegel aus dem Beleuchtungsstrahlengang ausgespiegelt und mittels einer Optik in eine Bildebene abgebildet. Der Beobachter verändert die Phoroptereinstellung so lange, bis das Netzhautbild in der Bildebene scharf erscheint. Der Patient kann durch Beobachten einer der Testmarken die Sehverbesserung verfolgen und kann so selbst an der optimalen Einstellung mitwirken.

Dieses Gerät hat den Nachteil, dass eine Korrektur nur in diskontinuierlichen Schritten und nur monokular möglich ist und dass durch den vor dem Patientenauge angeordneten Phoropter die bekannte Gerätemyopie auftritt.

Aus der US-PS 3 927 933 ist ein Gerät zur subjektiven und objektiven Refraktionsbestimmung bekannt, das als sogenanntes Freisichtgerät ausgebildet ist. Bei diesem Gerät wird eine Testmarke über zwei voneinander getrennte Projektionsstrahlengänge auf einen Hohlspiegel abgebildet, der in einer vorgegebenen Entfernung vom Probanden angebracht ist und von diesem beidäugig betrachtet wird. In beiden Projektionsstrahlengängen sind Einstellsysteme mit kontinuierlich veränderbarer sphärischer und astigmatischer Wirkung angeordnet, die solange verstellt werden, bis der Proband die Testmarke scharf sieht. Das von den Probandenaugen reflektierte Licht wird nach Reflexion am Hohlspiegel über einen teildurchlässigen Spiegel in zwei Beobachtungsstrahlengänge ausgespiegelt, welche dieselben Einstellsysteme enthalten wie die Projektionsstrahlengänge.

Dieses Gerät hat den Nachteil, dass der Hohlspiegel relativ gross sein muss, damit sich die Pupille der Probandenaugen auf das Testbild einstellen kann, und dass damit das ganze Gerät gross baut. Zur notwendigen Grösse des Gerätes trägt auch bei, dass infolge des grossen Abstandes zwischen den Probandenaugen und dem Hohlspiegel die Verschiebewege der Einstellsysteme gross sind.

Es ist nun die Aufgabe der vorliegenden Erfindung eine als Freisicht-Gerät ausgebildete Vorrichtung zur subjektiven und objektiven Refraktionsbestimmung zu schaffen, die besonders kompakt ist und sich durch einen einfachen Aufbau auszeichnet.

Diese Aufgabe wird bei einer Vorrichtung nach dem Oberbegriff des Anspruches 1 dadurch gelöst, dass vor jedem Probandenauge ein teildurchlässiger Spiegel zur Umlenkung des Projektionsstrahlenganges in das Auge und in Lichtrichtung gesehen vor diesem Spiegel und hinter dem Einstellsystem ein Linsensystem angeordnet ist, dessen dem Auge zugewandte Linse von der Augenpupille einen Abstand hat, der ein Mehrfaches der Brennweite des Linsensystems ist, und das zusammen mit dem Auge das Testzeichen auf den Augenhintergrund abbildet, und dass ein zur Ausspiegelung des Beobachtungsstrahlenganges dienender Spiegel in der Ebene des vom Linsensystem erzeugten Bildes der Augenpupille angeordnet ist.

Der Proband sieht durch die vor seinen Augen angeordneten teildurchlässigen Spiegel das dem Bild der Umgebung überlagerte helle Testzeichen. Um die bekannte Gerätemyopie zu vermeiden darf die Vorrichtung selbst für den Patienten nicht sichtbar sein, d.h. die Projektionsstrahlengänge sollten zweckmässig seitlich der Probandenaugen verlaufen. Dies macht eine Einspiegelung über weitere Spiegel und damit eine verhältnismässig grosse Schnittweite des zur Abbildung der Testmarke dienenden Linsensystems erforderlich. Um die Verschiebewege der Einstellelemente und damit auch die Geräteabmessung klein halten zu können, ist dieses System als Tele-System ausgebildet, dessen Schnittweite ein Mehrfaches der System-Brennweite ist.

Die relativ kleinen Geräteaustrittspupillen vor den Probandenaugen bedingen eine genaue Ausrichtung des Gerätes auf die Augenpupille. Dies wird durch die Anordnung des zur Ausspiegelung des Beobachtungsstrahlenganges dienenden Umlenkspiegel in der Ebene des vom Tele-System erzeugten Bildes der Augenpupille ermöglicht. Durch Beobachten dieses Spiegels und damit der Augenpupille ist die erforderliche Ausrichtung dann relativ einfach möglich. Dies gilt besonders, wenn der Umlenkspiegel so ausgebildet ist, dass er einen zentralen teildurchlässigen Bereich aufweist, der von einem, eine Marke tragenden spiegelnden Bereich umgeben ist.

Es ist vorteilhaft, den Testzeichen-Projektor so auszubilden, dass er das Testzeichen nach Unendlich abbildet. Damit kann in dem vom Projektor kommenden Parallelstrahlengang eine Aufteilung in zwei räumlich voneinander getrennte Projektionsstrahlengänge erfolgen, die zum Zwecke der Justierung und erforderlicher Einstellungen gegeneinander und relativ zum Projektor verschiebbar sind.

Um mit der Vorrichtung nach der Erfindung auch Nahprüfungen durchführen zu können, sind der Testzeichen-Projektor oder das Testzeichen oder die Einstellsysteme axial so verschiebbar, dass für den Probanden das Testzeichen in einer Entfernung von beispielsweise 400 mm erscheint, die der Nahprüfentfernung entspricht.

Da bei einer Nahprüfung die Blickrichtung gesenkt wird und die Augen eine Konvergenzstellung einnehmen, sind bei der Vorrichtung nach der Erfindung die vor den Probanden-Augen angeordneten Spiegel um den Augendrehpunkt schwenkbar. Durch Schwenken dieser Spiegel in Verbindung mit der entsprechenden Verschiebung des Testzeichens in die Nahprüfentfernung wird die zur Nahprüfung erforderliche Augenstellung erreicht. Dabei ist es vorteilhaft, die Spiegel und die Elemente zur Testzeichenverschiebung bewegungsmässig zu koppeln, um eine möglichst einfache Bedienung des Gerätes zu ermöglichen.

Es kann weiterhin vorteilhaft sein, die gesamte Vorrichtung zur Nahprüfung kippbar zu lagern.

Zur notwendigen Korrektur der Fehlsichtigkeit sind in beiden Projektions- und beiden Beobachtungsstrahlengängen Einstellsysteme zur kontinuierlichen Einstellung sphärischer und astigmatischer Wirkungen angeordnet. Diese Einstellsysteme sind sehr einfach aufgebaut und bestehen jeweils aus einer sphärischen und einer Stokesschen Linse, die gemeinsam axial verschiebbar sind.

Die Einstellsysteme in einem Projektions- und dem zugeordneten Beobachtungsstrahlengang sind jeweils bewegungsmässig gekoppelt.

Die beiden Beobachtungsstrahlengänge sind jeweils so ausgebildet, dass die von den Augen reflektierte Strahlung in eine Bildebene abgebildet wird. Diese Bildebene wird dann über ein bekanntes Beobachtungssystem, beispielsweise einen Binokulartubus, vom Beobachter betrachtet. Dieser kann unter ständiger Beobachtung durch Verschieben der Einstellsysteme in den Strahlengängen das Testzeichen auf der Netzhaut der Augen scharf abbilden. Der Patient beobachtet dabei in freier und unbehinderter Sicht das Testzeichen und kann die objektive Einstellung durch den Beobachter ständig verfolgen und auch subjektiv korrigieren. Dazu wird zweckmässig nach der objektiven Bestimmung eine subjektive Bestimmung vorgenommen, die der Proband auch selbst durchführen kann.

Mit der neuen Vorrichtung lässt sich also für beide Augen des Probanden unabhängig voneinander eine objektive und subjektive Refraktionsbestimmung durchführen, wobei der Proband freie, unbehinderte Sicht hat. Die Vorrichtung selbst ist kompakt und bedienungsfreundlich aufgebaut und ermöglicht sowohl eine Fern- als auch eine Nahprüfung.

Die Erfindung wird im folgenden anhand der Fig. 1 bis 4 der beigefügten Zeichnungen näher erläutert. Im einzelnen zeigen:

Fig. 1 die Anordnung der optischen Elemente bei einem Ausführungsbeispiel;

Fig. 2 einen der im Beispiel der Fig. 1 verwendeten Spiegel zur Ausspiegelung des Beobachtungsstrahlenganges in Draufsicht;

Fig. 3 ein Ausführungsbeispiel in perspektivischer Darstellung;

Fig. 4 ein anderes Ausführungsbeispiel, ebenfalls in perspektivischer Darstellung.

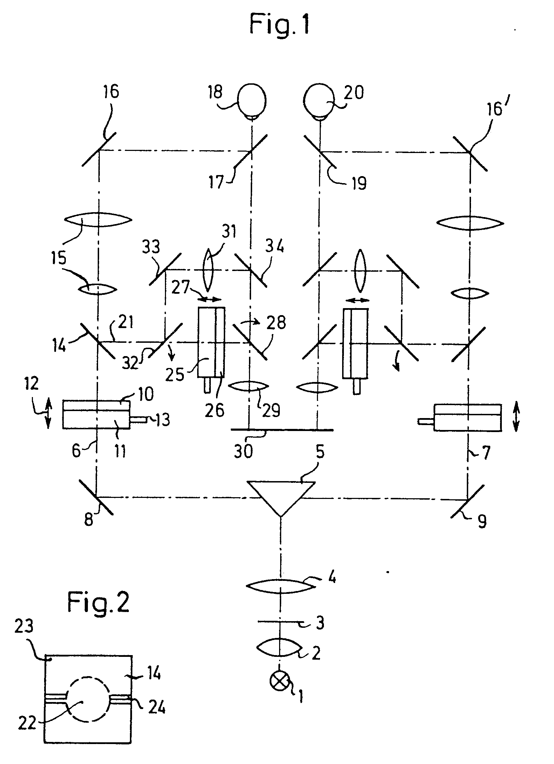

In Fig. 1 ist mit 1 eine Lichtquelle bezeichnet, die über einen Kondensor 2 eine Testmarke 3 beleuchtet. Ein Linsensystem 4 bildet die Testmarke 3 nach Unendlich ab. Im Parallelstrahlengang hinter dem Linsensystem 4 ist ein Prisma 5 angeordnet, das zur Aufteilung des Strahlenganges in zwei räumlich voneinander getrennte Projektionsstrahlengänge 6 und 7 dient, wobei die Spiegel 8 und 9 zur Strahlumlenkung dienen.

Die beiden Projektionsstrahlengänge 6 und 7 sind symmetrisch aufgebaut, so dass im folgenden nur der Strahlengang 6 erläutert wird. Die entsprechenden Elemente und Funktionen finden sich ebenso im Strahlengang 7.

Das vom Spiegel 8 in den Projektionsstrahlengang 6 umgelenkte Licht tritt zunächst durch ein Einstellsystem, das aus einer sphärischen Linse 10 und einer Stokesschen Linse 11 besteht, die gemeinsam axial verschiebbar sind, wie dies durch den Pfeil 12 angedeutet ist. Mittels des Bedienungsgliedes 13 kann die astigmatische Wirkung der Stokesschen Linse kontinuierlich verändert werden. Der dabei entstehende sphärische Anteil kann durch Verschieben des Einstellsystems 10, 11 kompensiert werden.

Die Testmarke 3 wird von der Linse 10, einem Linsensystem 15 und den Spiegeln 16, 17 auf die Netzhaut des zu prüfenden Auges 18 abgebildet. Ist dieses Auge normalsichtig, so wird der Test 3 von der Linse 10 in den projektorseitigen Brennpunkt des Linsensystems 15 abgebildet. Das Auge 18 bildet dann die vom System 15 nach Unendlich abgebildete Testmarke 3 scharf auf der Netzhaut ab, d.h. der Proband sieht die Testmarke scharf. Der vor dem Auge 18 angeordnete Spiegel 17 ist teildurchlässig, so dass der Proband völlig entspannt die Umgebung betrachten kann, der sich das helle Testbild überlagert.

Zur Refraktionsprüfung von Kindern können diesen beispielsweise bewegte Bilder gezeigt werden, welche eine Akkomodationsentspannung herbeiführen.

Ist das zu prüfende Auge 18 nicht normalsichtig, so wird die Linse 10 axial solange verschoben, bis die Testmarke 3 scharf auf der Netzhaut des Auges abgebildet wird. Die axiale Stellung der Linse 10 ist ein Mass für die sphärische Refraktion.

Astigmatische Fehlsichtigkeiten werden durch Betätigen der Stokesschen Linse 11 korrigiert. Sie wird, wie dargestellt, zusammen mit der Linse 10 axial verschoben, da eine Abstandsänderung zwischen den Linsen 10 und 11 zu einer Verfälschung der astigmatischen Korrektion führen würde.

Die Stellung des Bedienungsgliedes 13 ist ein Mass für den gesuchten astigmatischen Refraktionswert.

Die beiden Projektionsstrahlengänge 6 und 7 verlaufen seitlich von den Augen 18 und 20, um eine Gerätemyopie zu vermeiden. Dies bedingt einen grossen Abstand zwischen den Spiegeln 16 und 17. Um zu vermeiden, dass dadurch der Verschiebeweg für das Einstellsystem 10, 11 und damit das ganze Gerät gross wird, ist das Linsensystem 15 als Telesystem ausgebildet. Damit wird der Abstand der dem Auge zugewandten Linse von der Pupille des Auges 18 ein Mehrfaches der Brennweite des Systems 15. Es ist beispielsweise möglich, dem Linsensystem 15 eine Schnittweite von 200 mm bei einer Brennweite von 31 mm zu geben. Mit einer solchen Ausbildung entspricht ein Verschiebeweg des Einstellsystems 10, 11 von 1 mm etwa einer Änderung des Refraktionswertes um eine Dioptrie.

Das von der Netzhaut des Auges 18 reflektierte Licht wird über einen im Projektionsstrahlengang 6 angeordneten Spiegel 14 in den Beobachtungsstrahlengang 21 ausgespiegelt. Dieser Spiegel ist so angeordnet, dass das Linsensystem 15 die Pupille des Auges 18 in seine Ebene abbildet.

Der Spiegel 14 weist, wie Fig. 2 zeigt, einen zentralen teildurchlässigen Bereich 22 auf, der von einem spiegelnden Bereich 23 umgeben ist. Dieser Bereich 23 trägt eine Marke 24. Der Projektionsstrahlengang 6 verläuft durch den zentralen Bereich 23. Eine exakte Justierung der Gesamtvorrichtung ist erreicht, wenn der Beobachter die Augenpupille im Zentralbereich 23 liegen sieht und die Marke 24 symmetrisch dazu liegt.

Der Spiegel 14 ist ausklappbar. Damit kann die Austrittspupille des Projektionsstrahlenganges bei subjektiver Augenprüfung vergrössert werden.

Der Beobachtungsstrahlengang 21 enthält ein Einstellsystem 25, 26, das ebenso aufgebaut ist wie das Einstellsystem 10, 11 im Projektionsstrahlengang 6 und das, wie Pfeil 27 zeigt, axial verschiebbar ist. Die Verschiebung der Einstellsysteme 10, 11 und 25, 26 ist gekoppelt. Durch das Einstellsystem 25, 26 wird die Netzhaut des Auges 18 nach Unendlich abgebildet. Nach Umlenkung am klappbaren Spiegel 28 erzeugt ein Linsensystem 29 ein reelles Bild der Netzhaut in der Bildebene 30.

Parallel zum Einstellsystem 25, 26 ist eine Linse 31 angeordnet. Mittels eines klappbaren Spiegels 32 kann das vom Spiegel 14 kommende Licht über die Spiegel 33, 34 und die Linse 31 geführt werden. Dabei wird ein Bild der Pupille des Auges 18 in der Bildebene 30 erzeugt.

Die Bildebene 30 wird mittels eines hier nicht dargestellten Beobachtungssystems, beispielsweise eines Binokulartubus, beobachtet.

Es ist möglich eine Lichtquelle 1 zu verwenden, die infrarotes Licht erzeugt. In diesem Fall wird das in der Ebene 30 erzeugte Bild über einen Bildwandler sichtbar gemacht.

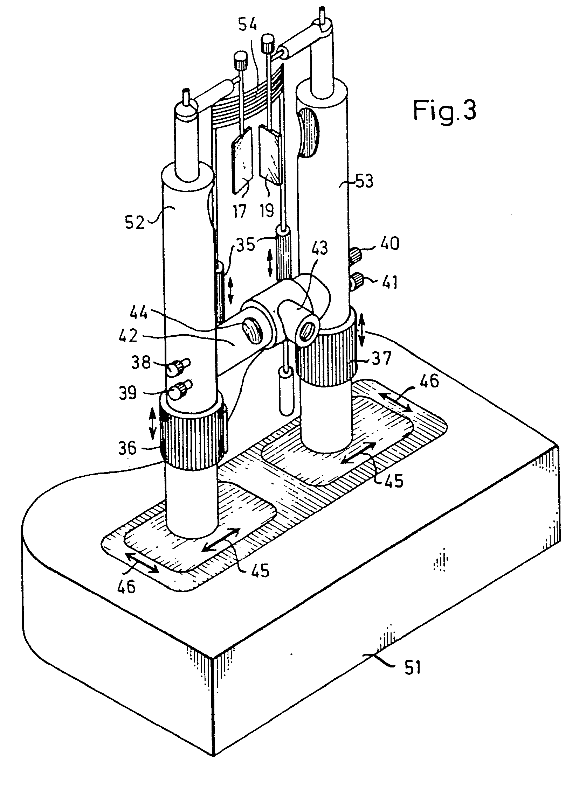

Die in ihrem optischen Aufbau in Fig. 1 dargestellte Vorrichtung kann in einem Gehäuse untergebracht sein, wie es in Fig. 3 gezeigt ist. Die Elemente 2 bis 5 sind in der Basis 51 angeordnet, während die Rohre 52 und 53 die Projektionsstrahlengänge 6 und 7 aufnehmen. Die Elemente 8 und 10 bis 16 sind im Rohr 52 angeordnet; die teildurchlässigen Spiegel 17, 19 werden von den Rohren 52, 53 getragen. Mit 54 ist eine Kopfstütze für den Probanden bezeichnet, die mittels der Ringe 35 höhenverstellbar ist. Zwei Ringe 36, 37 auf den Rohren 52, 53 dienen zur Höhenverstellung des Gerätes.

Die Betätigungsglieder 38, 40 dienen zur Axialverschiebung der Einstellsysteme 10, 11 und der damit gekoppelten Einstellsysteme im Beobachtungsstrahlengang 21. Die Glieder 39, 41 dienen zur Einstellung des Astigmatismus.

Die Beobachtungsstrahlengänge sind im Rohr 42 untergebracht, das aus zwei ineinander verschiebbaren Teilen besteht. Auf dem Rohr 42 ist verschiebbar ein Schlitten 43 angeordnet, der wahlweise in die Positionen gebracht werden kann, die eine Betrachtung der in der Bildebene 30 erzeugten Testmarken-Bilder ermöglicht. Das vom rechten Auge 18 erzeugte Testmarkenbild ist z.B. über die Öffnung 44 im Rohr 42 zu beobachten.

Die Wirkungsweise des Gerätes nach Fig. 1 und 2 ist folgende:

Nachdem der Proband seinen Platz eingenommen und Kopfstütze 54 sowie Gerät in der Höhe richtig eingestellt sind, wird durch Verschieben der Rohre 52, 53 auf der Basis 51 (Pfeile 45) die Pupillendistanz eingestellt.

Die richtige Bestimmung des Refraktionszustandes ist abhängig vom Hornhautscheitelabstand. Das Gerät ist beispielsweise auf einen Hornhautscheitelabstand von 16 mm geeicht. Zunächst wird mittels der Linse 31 auf den Ort des Spiegels 14 bzw. auf die Marke 24 fokussiert. Danach wird bei feststehender Linse 31 das Gerät in Richtung der Pfeile 46 solange verschoben, bis die Pupille des Auges zugleich mit der Marke 24 in der Bildebene 30 scharf erscheint. Damit ist der Hornhautscheitelabstand richtig eingestellt. In der Bildebene 30 ist eine, hier nicht dargestellte, zur Gerätepupille symmetrisch angeordnete Markierung vorgesehen, welche die Einstellung des Gerätes zu den Probanden-Augen nach Seite und Höhe ermöglicht.

Als nächster Schritt wird die objektive Refraktion vorgenommen. Dazu werden über die Betätigungsglieder 38, 39 die Einstellelemente 10, 11 und gekoppelt damit die Einstellelemente 25, 26 solange verschoben, bis die Testmarke 3 in der Bildebene scharf erscheint. Die Beobachtung erfolgt durch die Öffnung 44. Der Proband kann diese Einstellung selbst kontrollierend beobachten, wenn Licht im sichtbaren Spektralgebiet zur Projektion der Testmarke 3 verwendet wird.

Nach erfolgter Einstellung wird der Schlitten 43 nach rechts verschoben und durch Betätigen der Glieder 40, 41 wird solange eingestellt, bis die vom Auge 20 reflektierte Testmarke in der Bildebene 30 scharf erscheint.

Nach Abschluss der objektiven Refraktion kann der Proband selbst oder der Beobachter durch Betätigen der Glieder 38, 39, 40, 41 eine optimale Korrektur der Fehlsichtigkeit herbeiführen. Der Proband verstellt dazu das Gerät solange, bis er die Testmarke 3 mit beiden Augen optimal scharf sieht. Diese Einstellung kann unter ständiger Kontrolle durch Beobachten der Bildebene 30 erfolgen.

Das gezeigte Gerät ermöglicht auch eine Nahprüfung. Dazu wird z.B. der Projektor 1 bis 4 solange axial verschoben, bis die Testmarke 3 in der Ebene der Nahprüfentfernung (z.B. 400 mm) erscheint. Zugleich werden die Spiegel 17, 19 um den Augendrehpunkt geschwenkt, und zwar um den Winkel, um den sich das Auge beim Blick von der Ferne in die Nähe dreht. Nach Abschluss dieser Arbeiten wird in der bechriebenen Weise der erforderliche Nahzusatz objektiv und/oder subjektiv bestimmt.

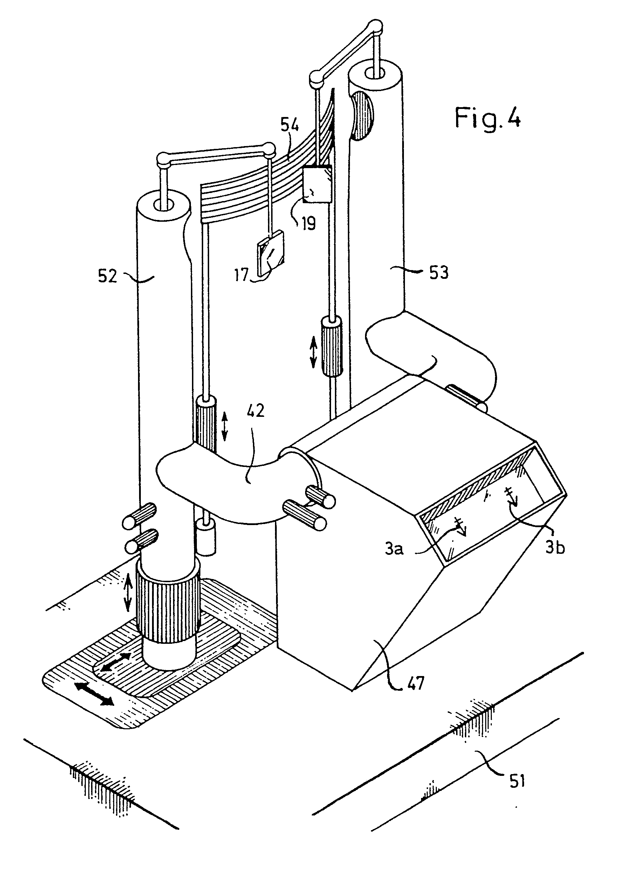

Fig. 4 zeigt ein Ausführungsbeispiel, bei dem anstelle der Beobachtungsvorrichtung 43 der Fig. 3 ein fest mit der Basis 51 verbundener Projektionszusatz 47 verwendet ist, der die Testmarken 3a, 3b auf einen Bildschirm projiziert. Ein solches Gerät findet vorteilhaft Anwendung, wenn zur Projektion der Testmarke 3 infrarotes Licht verwendet wird.

**Patentansprüche**

1. Vorrichtung zur subjektiven und objektiven Refraktionsbestimmung, bei welcher ein Testzeichen (3) über zwei voneinander getrennte Projektionsstrahlengänge (6, 7) in die Probandenaugen (18, 20) abgebildet wird und bei welcher das von den Augen reflektierte Licht aus den Projektionsstrahlengängen ausgespiegelt und einem Beobachter zugeleitet wird, wobei sowohl in den Projektionsstrahlengängen (6, 7) als auch in den Beobachtungsstrahlengängen (21) Einstellsysteme (10, 11 und 25, 26) mit kontinuierlich veränderbarer sphärischer und astigmatischer Wirkung angeordnet sind, dadurch gekennzeichnet, dass vor jedem Probandenauge (18, 20) ein teildurchlässiger Spiegel (17, 19) zur Umlenkung des Projektionsstrahlenganges (6, 7) in das Auge und in Lichtrichtung gesehen vor diesem Spiegel und hinter dem Einstellsystem (10, 11) ein Linsensystem (15) angeordnet ist, dessen dem Auge zugewandte Linse von der Augenpupille einen Abstand hat, der ein Mehrfaches der Brennweite des Linsensystems beträgt, und das zusammen mit dem Auge das Testzeichen (3) auf der Netzhaut abbildet, und dass ein zur Ausspiegelung des Beobachtungsstrahlenganges (21) dienender Spiegel (14) in der Ebene des vom Linsensystem (15) erzeugten Bildes der Augenpupille angeordnet ist.

2. Vorrichtung nach Anspruch 1, dadurch gekennzeichnet, dass ein Testzeichenprojektor (1, 2, 3, 4) zur wahlweisen Abbildung der Testmarke (3) nach Unendlich oder in eine vorgegebene Entfernung und Reflexionselemente (5) zur Aufteilung des vom Projektor kommenden Strahlenganges in zwei räumlich voneinander getrennte, in seitlichem Abstand von den Probandenaugen (18, 20) verlaufenden Strahlengänge (6, 7) vorgesehen sind.

3. Vorrichtung nach Anspruch 2, dadurch gekennzeichnet, dass in jedem Projektionsstrahlengang (6, 7) die Einstellsysteme (10, 11) zur Einstellung sphärischer und astigmatischer Wirkungen gemeinsam axial verschiebbar angeordnet sind.

4. Vorrichtung nach Anspruch 1, dadurch gekennzeichnet, dass der zur Ausspiegelung des Beobachtungsstrahlenganges dienende Spiegel (14) einen zentralen teildurchlässigen Bereich (22) aufweist, der von einem, eine Marke (24) tragenden spiegelnden Bereich (23) umgeben ist.

5. Vorrichtung nach Anspruch 3, dadurch gekennzeichnet, dass in jedem Beobachtungsstrahlengang (21) die Einstellsysteme (25, 26) zur Einstellung sphärischer und astigmatischer Wirkungen gemeinsam axial verschiebbar angeordnet und bezüglich dieser Verschiebung mit den Einstellsystemen (10, 11) im zugeordneten Projektionsstrahlengang gekoppelt sind.

6. Vorrichtung nach Anspruch 5, dadurch gekennzeichnet, dass in Lichtrichtung gesehen hinter den Einstellsystemen (25, 26) im Beobachtungsstrahlengang ein erstes Linsensystem (29) zur Abbildung des Augenhintergrundes in eine Bildebene (30) angeordnet ist.

7. Vorrichtung nach Anspruch 4 und 6, dadurch gekennzeichnet, dass parallel zu den Einstellsystemen (25, 26) und zum ersten Linsensystem (29) in jedem Beobachtungsstrahlengang (21) ein wahlweise einschaltbares zweites Abbildungssystem (31) angeordnet ist.

8. Vorrichtung nach Anspruch 1, dadurch gekennzeichnet, dass die vor den Probandenaugen (18, 20) angeordneten teildurchlässigen Spiegel um den jeweiligen Augendrehpunkt schwenkbar sind.

9. Vorrichtung nach einem der Ansprüche 1 bis 8, dadurch gekennzeichnet, dass der Testmarkenprojektor (1, 2, 3, 4) und die zur Strahlteilung dienenden Reflexionselemente (5) in einem Basisgehäuse (51) untergebracht sind, auf dem zwei, die Elemente der Projektionsstrahlengänge (6, 7) enthaltende zylinderförmige Gehäuse (52, 53) zur Einstellung der Pupillendistanz und des Hornhautscheitelabstandes verschiebbar angeordnet sind.

10. Vorrichtung nach Anspruch 9, dadurch gekennzeichnet, dass das Basisgehäuse (51) zusammen mit den zylinderförmigen Gehäusen (52, 53) zur Nahprüfung kippbar ist.

**Claims**

1. Apparatus for the subjective and objective determination of refraction in which a test mark (3) is focused into the eyes (18, 20) of a test subject via two projection rays paths (6, 7) which are spaced from each other and in which the light reflected by the eyes is reflected out of the projection ray paths and fed to an observer, and in which adjustment systems (10, 11 and 25, 26) of continuously variable spherical and astigmatic effect are arranged both in the projection ray paths (6, 7) and in the observation ray paths (21), characterized in that in front of each eye (18, 20) of the test subject there is arranged a partially transmitting mirror (17, 19) for deflecting the projection ray path (6, 7) into the eye, and in front of said mirror and behind the adjustment system (10, 11), as seen in the direction of the light, there is arranged a lens system (15) the lens of which facing the eye is at a distance from the pupil of the eye which is a multiple of the focal length of this lens system and which, together with the eye, focuses the test mark (3) on the retina of the eye, and by the fact that a mirror (14) which serves for reflecting out the observation ray path (21) is arranged in the plane of the image of the pupil of the eye produced by the lens system (15).

2. Apparatus according to claim 1, characterized in that there are provided a test-mark projector (1, 2, 3, 4) for the optional focusing of a test mark (3) at infinity or at a predetermined distance, and reflection means (5) for dividing the ray path coming from the projector into two ray paths (6, 7) arranged separated from each other in space and extending at a lateral distance from the eyes (18, 20) of the test subject.

3. Apparatus according to claim 2, characterized in that within each projection ray path (6, 7) the adjustment systems (10, 11) for adjusting spherical and astigmatic effects are arranged for joint axial displacement.

4. Apparatus according to claim 1, characterized in that the mirror (14) which serves for reflecting out the observation ray path contains a central partially transmitting region (22) which is surrounded by a reflecting region (23) which bears a mark (24).

5. Apparatus according to claim 3, characterized in that in each observation ray path (21) adjustment systems (25, 26) for adjusting spherical and astigmatic effects are arranged for joint axial displacement and are coupled with respect to such displacement, with said adjustment systems (10, 11) in the associated projection ray path.

6. Apparatus according to claim 5, characterized in that behind said adjustment systems (25, 26), in the observation ray path as seen in the direction of the light a first lens system (29) for imaging the fundus of the eye in an image plane (30) is arranged.

7. Apparatus according to claims 4 and 6, characterized in that parallel to the adjustment systems (25, 26) and to the first lens system (29) an optionally interposable second focusing system (31) is arranged in each observation ray path (21).

8. Apparatus according to claim 1, characterized in that said partially transmitting mirrors arranged in front of the eyes (18, 20) of the test subject are swingable around the center of rotation of the corresponding eye.

9. Apparatus according to one of claims 1 to 8, characterized in that the test-mark projector (1, 2, 3, 4) and the reflection elements (5) for dividing the ray path are arranged in a base housing (51), on which two cylindrical housings (52, 53) which contain the elements of the projection ray paths (6, 7) are displaceable mounted for adjusting the interpupillary distance and the corneal vertex distance.

10. Apparatus according to claim 9, characterized in that said base housing (51) is tiltable together with said cylindrical housings (52, 53) for near-vision examination.

**Revendications**

1. Dispositif pour la détermination subjective et objective de réfraction, dans lequel une image d'un signe de référence (3) est formée par l'intermédiaire de deux faisceaux de rayons de projection (6, 7) séparés l'un de l'autre dans les yeux (18, 20) du patient et dans lequel la lumière réfléchie par les yeux est déviée à partir des faisceaux de rayons de projection (6, 7) et est transmise à un observateur, des systèmes de réglage (10, 11 et 25, 26) à effet sphérique et astigmatique modifiable de façon continue étant disposés aussi bien dans les faisceaux des rayons de projection (6, 7) qu'également dans les faisceaux des rayons d'observation (21), caractérisé en ce qu'il est prévu devant chaque œil de patient (18, 20) un miroir partiellement transparent (17, 19) pour dévier le faisceau des rayons de projection (6, 7) dans l'œil et, en considérant la direction de la lumière devant ce miroir et en arrière du système de réglage (10, 11), un système à lentilles (15), dont la lentille tournée vers l'œil est espacée de la pupille de l'œil d'une distance qui est égale à un multiple de la distance focale du système à lentilles et qui forme, en coopération avec l'œil, une image du signe de référence (3) sur la rétine, et en ce qu'un miroir (14) servant à la déviation à partir du faisceau des rayons d'observation (21) est placé dans le plan de l'image de la pupille d'œil produite par le système à lentilles (15).

2. Dispositif selon la revendication 1, caractérisé en ce qu'il est prévu un projecteur de signe de référence (1, 2, 3, 4) pour former sélectivement une image du repère de référence (3) à l'infini ou bien à une distance d'éloignement prédéterminée, et des éléments réfléchissants (5) pour diviser le faisceau de rayons provenant du projecteur en deux faisceaux de rayons (6, 7) séparés spatialement l'un de l'autre et disposés avec espacement latéral par rapport aux yeux de patient (18, 20).

3. Dispositif selon la revendication 2, caractérisé en ce que, dans chaque trajet de faisceau de rayons de projection (6, 7), les systèmes de réglage (10, 11) servant à régler des effets sphériques et astigmatiques sont disposés de façon à être déplaçables axialement ensemble.

4. Dispositif selon la revendication 1, caractérisé en ce que le miroir (14) servant à la déviation du faisceau de rayons d'observation comporte une zone centrale partiellement transparente (22) qui est entourée par une zone réfléchissante (23) portant un repère (24).

5. Dispositif selon la revendication 3, caractérisé en ce que, dans chaque trajet de faisceau de rayons d'observation (21), les systèmes de réglage (25, 26) servant à régler des effets sphériques et astigmatiques sont disposés de façon à être déplaçables axialement ensemble et sont couplés, en ce qui concerne ce déplacement, avec les systèmes de réglage (10, 11) placés dans le faisceau associé de rayons de projection.

6. Dispositif selon la revendication 5, caractérisé en ce que, en considérant la direction de la lumière, il est prévu, en arrière des systèmes de réglage (25, 26) dans le faisceau de rayons d'observation un premier système à lentilles (29) pour former une image de l'arrière plan de l'œil dans un plan-image (30).

7. Dispositif selon l'une des revendications 4 et 6, caractérisé en ce qu'un second système de formation d'image (31) enclenchable sélectivement est disposé, parallèlement au système de réglage (25, 26) et au premier système à lentilles (29), dans chaque faisceau de rayons d'observation (21).

8. Dispositif selon la revendication 1, caractérisé en ce que des miroirs partiellement transparents placés devant les yeux de patient (18, 20) peuvent pivoter autour du centre de rotation correspondant des yeux.

9. Dispositif selon l'une des revendications 1 à 8, caractérisé en ce que le projecteur de repère de référence (1, 2, 3, 4) et les éléments réfléchissants (5) servant à la division des rayons sont logés dans un carter de base (51), sur lequel sont disposés deux boîtiers de forme cylindrique (52, 53) contenant les éléments des faisceaux de rayons de projection (6, 7) en étant déplaçables pour le réglage de la distance de pupille et de l'espacement de sommet de cornée.

10. Dispositif selon la revendication 9, caractérisé en ce que le carter de base (51) peut être basculé en même temps que les boîtiers de forme cylindrique (52, 53) pour un contrôle de point proximum.

# Fig.1

# Fig.2

Fig.3

Fig.4